# EUROPÄISCHE PATENTSCHRIFT

(11) **EP 3 344 988 B1**
(45) Veröffentlichungstag und Bekanntmachung des Hinweises auf die Patenterteilung: **31.07.2019**
(21) Anmeldenummer: 16758149.5
(22) Anmeldetag: 26.08.2016
(51) Int. Cl.: G01N 33/15, G01N 33/00

(54) **VERFAHREN UND VORRICHTUNG ZUR PRÜFUNG VON TABLETTEN**
METHOD AND DEVICE FOR TESTING TABLETS
PROCÉDÉ ET DISPOSITIF POUR CONTRÔLER DES COMPRIMÉS

(30) Priorität: 01.09.2015 DE 102015114600
(43) Veröffentlichungstag der Anmeldung: 11.07.2018
(73) Patentinhaber: ERWEKA GmbH, 63150 Heusenstamm (DE)
(72) Erfinder: MÜLLER, Werner Georg, 63150 Heusentamm (DE); BOZKURT, Levent, 63150 Heusenstamm (DE)
(74) Vertreter: Dr. Meyer-Dulheuer & Partners LLP
(86) Internationale Anmeldenummer: PCT/EP2016/070229
(87) Internationale Veröffentlichungsnummer: WO 2017/036980

(56) Entgegenhaltungen:
- EP-A2- 1 445 217
- DE-U1-202015 101 878

## Beschreibung

Gegenstand der vorliegenden Erfindung sind eine Vorrichtung und ein Verfahren zur Prüfung von Tabletten. Die Vorrichtung zur Prüfung von Tabletten umfasst eine Prüfkammer, die eine Bruchbacke und eine ihr gegenüberliegende Gegenbacke aufweist. Die Vorrichtung verfügt über einen beweglichen Blechstreifen, der dazu eingerichtet ist, eine Tablette zur Prüfung zu positionieren. Die Längsachse des beweglichen Blechstreifens ist in einem Winkel von unter 90° zur Längsachse der Bewegungsrichtung der Bruchbacke ausgerichtet und die Bewegungsrichtung des Blechstreifens entspricht der Längsachse des Blechstreifens.

Im Rahmen der Qualitätskontrolle von Tabletten ist es notwendig, diese auf ihre Eigenschaften wie Länge, Breite, Bruchfestigkeit und Gewicht zu überprüfen. Das Deutsche Arzneibuch enthält die hierfür relevanten gesetzlichen Bestimmungen. Dieser Vorgang sollte nach Möglichkeit automatisch vonstattengehen, sodass eine Vielzahl von Tabletten innerhalb kurzer Zeit überprüft werden kann. Zudem muss gewährleistet werden, dass Tabletten unterschiedlicher Formen und Größen richtig positioniert werden können, um eine fehlerfreie Messung zu ermöglichen. Der Stand der Technik kennt mehrere Vorrichtungen und Verfahren, die hierfür geeignet sind.

Die Deutsche Patentschrift DE 197 33 436 C2 beschreibt ein Tablettentestgerät zur Prüfung von Oblongtabletten. Hierbei werden die zu prüfenden Tabletten durch eine Zufuhreinrichtung zunächst einer Waage zugeführt und von dieser anschließend auf eine Transporteinrichtung verbracht. Auf der Transporteinrichtung werden die Tabletten hierbei in einer definierten Position abgelegt, sodass weitere Tests stattfinden können. Die korrekte Ausrichtung der Tablette bei Ablage auf der Transporteinrichtung wird hierbei dadurch bewirkt, dass die Waagschale einen Boden in Form einer Rinne aufweist. Dieser sorgt dafür, dass die Oblong-Tabletten eine entsprechende Ausrichtung erfahren. Darüber hinaus kann sich auf der Transporteinrichtung ein Stolperstein, beispielsweise ein Keil, befinden, der für den Fall vorgesehen ist, dass die Tablette mit ihrer Kopfseite auf die Transportvorrichtung fällt. In diesem Fall sorgt der Stolperstein dafür, dass die Tablette auf ihre lange Seite kippt. Gemäß dieser Erfindung wird eine korrekte Ausrichtung einer Tablette bewirkt, jedoch ist die Tablette bei ihrer Übergabe durch die Rinne nicht unerheblichen Kräften ausgesetzt, die zu ihrer Beschädigung führen könnten.

Die Deutsche Patentschrift DE 10 2006 004 215 B4 offenbart einen Bruchfestigkeitstester für Tabletten unterschiedlicher Form und Größe. Um die Ausrichtung der Tabletten zur bewerkstelligen, werden hier zwei gegenläufig angetriebene, nebeneinander angeordnete Zentrierrollen eingesetzt. Wird eine Oblong-Tablette auf den Rollen platziert, so sorgt deren Rotation dafür, dass sich die Oblong-Tablette in Längsrichtung zwischen den Rollen ausrichtet. Anschließend kann eine Überprüfung der Festigkeit durch eine Bruchbacke erfolgen. Um die Ausrichtbarkeit von Tabletten unterschiedlicher Form und Größe zu gewährleisten, ist die Vorrichtung mit einem schwenkbaren Positionierer ausgestattet, der die Rollen unterschiedlich ausrichten kann. Die Ausrichtung geht schnell vonstatten, die Apparatur könnte aufgrund der Verwendung von Rollen nach einem erfolgten Bruchtest allerdings schwer zu reinigen sein.

Die Gebrauchsmusterschrift DE 298 24 199 U1 offenbart ein System zur Durchführung von Härtetests an Prüfkörpern, die einen alternativen Ansatz verfolgt. Hierbei wird die Tablette auf einem Prüftisch platziert, aber daraufhin wird keine Ausrichtung der Tablette durchgeführt. Stattdessen wird durch eine Vorrichtung zur Lageerkennung die Ausrichtung des Prüfkörpers festgestellt. Daraufhin werden ein Druckkolben sowie ein Gegenlager, die zum Härtetest der Tablette dienen, entsprechend ausgerichtet, damit die Überprüfung der Tablette erfolgen kann. Diese Erfindung setzt jedoch einen komplizierten und kostspieligen Aufbau voraus, der Mittel zur Bilderkennung umfassen muss.

Die PCT-Veröffentlichungsschrift WO 2013/061223 A2 offenbart ein Verfahren und eine Vorrichtung zur Überprüfung von Tabletten, das die Tabletten unter Zuhilfenahme einer schwenkbaren Wippe ausrichtet. Die Tablette wird hierbei auf die zunächst waagerechte Wippe verbracht, die nun eine Schwenkbewegung durchführt. Durch die Wirkung der Schwerkraft und der Schwenkbewegung wird bewirkt, dass die Tablette sich an einer Positionierungsfläche ausrichtet. Die Positionierungsfläche hat hierbei eine konkave Form, die entlang der durch die Bewegung der Wippe definierten Form verläuft. Um die korrekte Ausrichtung der Tablette zu unterstützen, kann die schwenkbare Wippe zusätzliche in einem Winkel zur Positionierungsfläche geneigt sein, oder aber eine zusätzliche Einrichtung auf der Seite gegenüberliegend der Positionierungsfläche besitzen, welche eine Vorausrichtung der Tablette vornimmt. Nach erfolgter Positionierung und Rückkehr der Wippe in eine waagerechte Position erfolgt die Überprüfung der Tablette. Da die Positionierung hier unter Einwirkung der Schwerkraft geschieht und die Wippbewegung mit einer ausreichenden Geschwindigkeit durchgeführt werden muss, kann auch hier nicht ausgeschlossen werden, dass die auf die Tablette wirkenden Kräfte bei der Ausrichtung zu hoch sein könnten.

Die Gebrauchsmusterschrift DE 20 2015 101 878 U1 offenbart ein Härteprüfgerät für ein Oblong mit einer Fest- und einer Pressbacke, bei der ein Endlosband zum Positionieren des Oblongs den Boden einer Bruchkammer bildet. Die Ausrichtung des Endlosbandes wird mittels einer Verstellvorrichtung in 45°-Schritten in der Bruchkammerbodenebene gedreht.

Die Europäische Veröffentlichungsschrift EP 1 445 217 A2 offenbart einen drehbaren Transportstern mit einer Härteteststation, bei der Oblongs von Kammerwänden des Transportsterns mitgenommen und in die Härteteststation geschoben werden. Aufgabe der vorliegenden Erfindung war es daher, eine Vorrichtung und ein Verfahren zur Überprüfung von Tabletten bereitzustellen, das die Tablette vor ihrer Überprüfung in einer schonenden, schnellen und einfach zu implementierenden Weise ausrichtet, wobei auch eine problemlose Reinigbarkeit gewährleistet sein soll.

Gelöst wird die Aufgabe durch eine Vorrichtung gemäß Anspruch 1 und ein Verfahren gemäß Anspruch 10.

In der Prüfkammer ist vorzugsweise ein Widerlager vorgesehen, das die Prüfkammer begrenzt und sich von der Gegenbacke in Richtung der Bruchbacke erstreckt, wobei das Widerlager im Wesentlichen in einem rechten Winkel zu der Gegenbacke angeordnet ist. In einer besonders bevorzugten Ausführungsform ist das Widerlager als Teil eines Mitnehmers ausgestaltet. Bei dem Mitnehmer handelt es sich vorzugsweise um einen Transportstern.

Bei der Tablette handelt es sich vorzugsweise um eine Oblong-Tablette, die sich aufgrund ihrer Form ihrer Länge nach an der entsprechenden Positionierungsfläche (Gegenbacke oder Widerlager) ausrichtet. Es ist jedoch auch möglich, Tabletten anderer Form zu positionieren, beispielsweise runde Tabletten. Hierbei führt der Vorgang lediglich dazu, dass die Tablette an die Positionierungsfläche herangeführt wird, sodass eine Prüfung durchgeführt werden kann. Der Prüfvorgang an sich läuft ohne menschliches Zutun ab. Vorzugsweise verfügt die Vorrichtung über eine Rechenanlage, die nicht nur die Bewegungsabläufe steuert, sondern auch die gemessenen Werte speichert.

Bei einer weiteren vorteilhaften Ausgestaltungsform ist die Tablette entlang des Widerlagers durch Bewegung des Blechstreifens in eine Richtung positionierbar ist. Zusätzlich ist die Tablette entlang der Gegenbacke durch Bewegung des Blechstreifens in die entgegengesetzte Richtung positionierbar.

Erfindungsgemäß ist weiterhin bevorzugt, wenn ein Teil des Blechstreifens einen Boden der Prüfkammer bildet. Wenn der Mitnehmer die Tablette in die Prüfkammer befördert, kommt die Tablette automatisch auf dem Blechstreifen zu liegen und kann entsprechend für die weiteren Prüfungsschritte ausgerichtet werden.

Um eine bessere Positionierung der Tablette entlang den Positionierungsflächen zu ermöglichen, können die Positionierungsflächen (Gegenbacke und Widerlager) eine besondere Form aufweisen. Erfindungsgemäß wird eine ebene Form als besonders vorteilhaft angesehen. Es ist jedoch auch möglich, die Positionierungsflächen in einer konkaven Form auszugestalten. Es ist außerdem möglich, die Positionierungsflächen mit einer aufgerauten Oberfläche oder einem speziellen Muster zu versehen, welche eine bessere Ausrichtung der Tabletten erlauben.

In einer besonders bevorzugten Ausgestaltungsform der vorliegenden Erfindung ist die Bruchbacke dazu geeignet, den Durchmesser der Tablette zu messen, die entlang der Gegenbacke oder des Widerlagers ausgerichtet ist, wobei der Durchmesser in Richtung der Bewegungsrichtung der Bruchbacke gemessen wird. Handelt es sich bei der Tablette um eine Oblong-Tablette, so wird zunächst die Breite der Tablette gemessen und anschließend in einer zweiten Position die Länge der Tablette.

Bevorzugt ist die Bruchbacke weiterhin dazu geeignet, eine Überprüfung der Bruchhärte der Tablette durchzuführen, die entlang der Gegenbacke oder des Widerlagers ausgerichtet ist.

In einer weiteren vorteilhaften Ausgestaltung der Erfindung beträgt der Winkel zwischen der Längsachse des beweglichen Blechstreifens und der Längsachse der Bewegungsrichtung der Bruchbacke zwischen 20° und 60°, vorzugsweise zwischen 40° und 50°. Der Winkel ist derart gewählt, dass die zu prüfende Tablette bei Bewegung des Blechstreifens entlang der Gegenbacke und entlang des Widerlagers zügig ausgerichtet werden kann.

In einer besonders bevorzugten Ausführungsform ist eine Verstellvorrichtung vorgesehen, auf der der Blechstreifen angeordnet ist, wobei mittels der Verstellvorrichtung der Winkel zwischen der Längsachse des beweglichen Blechstreifens und der Längsachse der Bewegungsrichtung der Bruchbacke verstellbar ist. Somit kann der Winkel zwischen der Längsachse des beweglichen Blechstreifens und der Längsachse der Bewegungsrichtung der Bruchbacke auf bestimmte Formen von Tabletten oder unterschiedlich große Tabletten angepasst werden. Dies ermöglicht ein präzises Ausrichten unterschiedlicher Tabletten.

Außerdem ist erfindungsgemäß ein Mitnehmer vorgesehen, der das Tablettenmaterial nach erfolgter Prüfung aus der Prüfkammer schiebt. Der Mitnehmer kann somit sowohl die Tablette in die Prüfkammer transportieren, als auch nach erfolgter Prüfung die Tablette oder Tablettenbruchstücke aus der Prüfkammer rausschieben. Es ist erfindungsgemäß bevorzugt, wenn das Widerlager Teil des Mitnehmers ist.

Zusätzlich ist ein Verfahren zur Prüfung von Tabletten gemäß Anspruch 10 Gegenstand der vorliegenden Erfindung.

Die Ausrichtung der Tablette erfolgt dadurch, dass diese durch die diagonale Bewegung des Blechstreifens in Kontakt mit einer Positionierungsfläche kommt und an dieser Positionierungsfläche aufgrund der Bewegung des Blechstreifens entlang ihrer Längsachse ausgerichtet wird. Anschließend findet die Prüfung mindestens eines Parameters der Tablette statt.

Vorzugsweise wird zur Prüfung der Tablette in einer ersten Position diese dadurch positioniert, dass der bewegliche Blechstreifen in eine ersten Richtung bewegt wird, sodass die Tablette entlang einer Gegenbacke ausgerichtet wird. Um eine weitere Prüfung durchzuführen, wird die Tablette zur Prüfung in einer weiteren Position dadurch positioniert, dass der bewegliche Blechstreifen in die entgegengesetzte Richtung bewegt wird, sodass die Tablette an das Widerlager trifft und entlang des Widerlagers ausgerichtet wird. Handelt es sich bei der Tablette um eine Oblong-Tablette, so wird zunächst in einer ersten Position die Breite der Tablette gemessen und anschließend in einer zweiten Position die Länge. Nach erfolgter Längenmessung erfolgt der Bruchtest in der derselben Position.

Ein beispielhaftes Verfahren wird wie folgt durchgeführt:
1. Eine Tablette, vorzugsweise eine Oblong-Tablette, wird auf den Blechstreifen befördert.
2. Die Tablette wird durch Bewegung des Blechstreifens in eine Richtung an einer ersten Positionierungsfläche (Gegenbacke) ausgerichtet.
3. Eine Prüfung der Breite der Tablette wird durchgeführt.
4. Die Tablette wird durch die Bewegung des Blechstreifens in die entgegengesetzte Bewegungsrichtung an einer weiteren Positionierungsfläche (Widerlager) ausgerichtet.
5. Eine Prüfung der Länge der Tablette wird durchgeführt.
6. Ein Bruchtest der Tablette wird durchgeführt.
7. Die Überreste der Tablette werden von der Prüfkammer entfernt.

Abhängig von den angestrebten Prüfzielen ist aber auch alternativ ein anderer Ablauf des Verfahrens möglich: so beispielsweise eine Variante, bei der nur eine Messung der Durchmesser, aber kein Bruchtest durchgeführt wird, oder nur ein Bruchtest ohne vorherige Durchmessermessung. Der Begriff Durchmessermessung umfasst im Sinne der vorliegenden Erfindung eine Breiten- und/oder Längenmessung der Tablette.

Die Prüfung der Tablette wird erfindungsgemäß durch eine Bruchbacke durchgeführt. Die Bruchbacke vollzieht eine Bewegung in Richtung der Gegenbacke und wird durch einen Motor angetrieben. Die Bruchbacke ist dazu ausgelegt, den Durchmesser der Tablette sowie deren Bruchhärte zu überprüfen. Die Überprüfung der Dimensionen erfolgt hierbei dadurch, dass die Bruchbacke so lange in Richtung der Gegenbacke bewegt wird, bis eine definierte Gegenkraft auftritt. Diese Gegenkraft zeigt auf, dass die Bruchbacke auf die Tablette getroffen ist. Der zurückgelegte Weg der Bruchbacke wird gespeichert, wobei optional hieraus direkt der Durchmesser der Tablette errechnet wird. Der Durchmesser der Tablette berechnet sich aus dem maximal zurücklegbaren Weg zwischen der Ausgangsposition der Bruchbacke und dem Widerlager abzüglich des tatsächlich zurückgelegten Wegs der Bruchbacke.

Die Messung des Durchmessers kann entweder erfolgen, nachdem die Tablette entlang der Gegenbacke positioniert worden ist, oder aber nachdem die Tablette entlang des Widerlagers ausgerichtet worden ist. Ist eine Positionierung entlang der Gegenbacke durchgeführt worden, so erfolgt durch die Bruchbacke eine Messung des Breitendurchmessers. Ist eine Positionierung entlang des Widerlagers durchgeführt worden, so erfolgt durch die Bruchbacke eine Messung des Längsdurchmessers der Tablette.

Vorzugsweise ist die Bruchbacke zusätzlich dazu eingerichtet, eine Messung der Bruchhärte der Tablette durchzuführen. Zu diesem Zweck wird die Bruchbacke so lange in Richtung der Gegenbacke bewegt, bis auf die Bruchbacke eine definierte Gegenkraft auftritt. Dies zeigt an, dass die Bruchbacke die Tablette erreicht hat. Daraufhin wird die durch die Bruchbacke angelegte Kraft so lange gesteigert, bis die Tablette zerbricht. Die durch die Bruchbacke angelegte Kraft, die notwendig war, um einen Bruch der Tablette herbeizuführen, wird hierbei durch die integrierte Rechenanlage der Vorrichtung gespeichert. Die Messung der Bruchhärte kann nur erfolgen, wenn die Tablette zuvor an einer Positionierungsfläche ausgerichtet worden ist. Ist die Ausrichtung an der Gegenbacke durchgeführt worden, so erfolgt eine Prüfung der Bruchhärte bezogen auf die kurze Seite (Breite) der Tablette. Ist die Ausrichtung am Widerlager durchgeführt worden, so erfolgt eine Prüfung der Bruchhärte bezogen auf die lange Seite (Länge) der Tablette.

Die Tablette beziehungsweise deren Überreste werden nach erfolgter Prüfung durch einen Mitnehmer von der Prüfkammer geschoben. Der Mitnehmer führt in einer vorteilhaften Variante der Erfindung eine Bewegung in Richtung des Widerlagers aus. Diese Bewegung wird durch Motorkraft gestützt. Auch hier erfolgt die Steuerung des Vorgangs durch die in die Prüfungsvorrichtung intergierte Recheneinheit. Besonders bevorzugt ist eine Ausführungsform, bei der der Mitnehmer selbst das Widerlager ausbildet.

Die erfindungsgemäße Vorrichtung und das erfindungsgemäße Verfahren werden nachfolgend anhand der Figuren 1 bis 3 näher erläutert, wobei
Fig. 1 eine Draufsicht auf eine erfindungsgemäße Prüfkammer ohne Abdeckung (Mitnehmer) zeigt,
Fig. 2 eine Draufsicht auf eine erfindungsgemäße Vorrichtung mit einer zu prüfenden Oblong-Tablette zeigt, und
Fig. 3.1 bis 3.9 einen möglichen Ablauf der einzelnen Verfahrensschritte des erfindungsgemäßen Verfahrens zur Prüfung einer Oblong-Tablette zeigen.

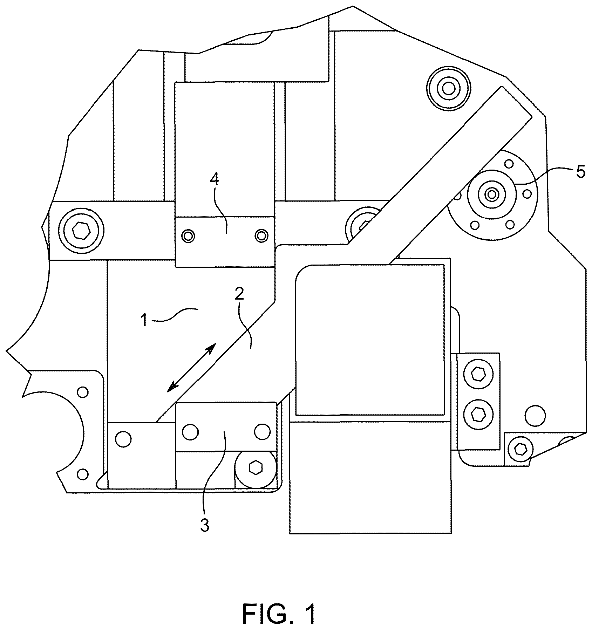

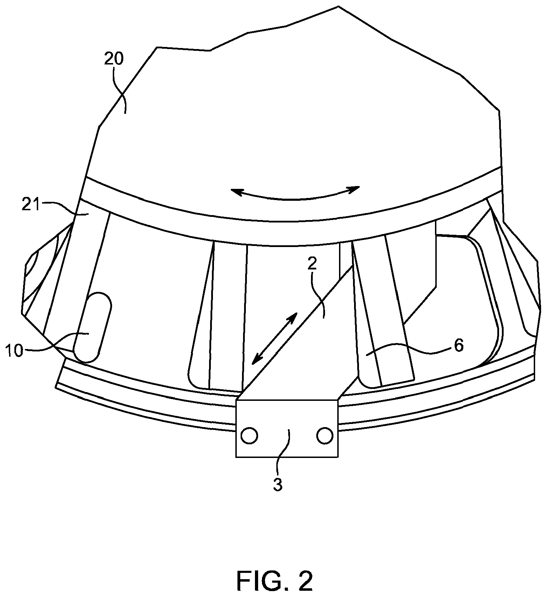

Wie in **Fig. 1** und **Fig. 2** dargestellt, umfasst die erfindungsgemäße Vorrichtung eine Prüfkammer (1), die eine Bruchbacke (4) und eine ihr gegenüberliegende Gegenbacke (3) enthält. Die Vorrichtung verfügt über einen beweglichen Blechstreifen (2), der dazu eingerichtet ist, eine Tablette zur Prüfung zu positionieren. Die Längsachse des beweglichen Blechstreifens (2) ist in einem Winkel von unter 90° zur Längsachse der Bewegungsrichtung der Bruchbacke (4) ausgerichtet und die Bewegungsrichtung des Blechstreifens (2) entspricht der Längsachse des Blechstreifens (2). Die Bewegungsrichtung des Blechstreifens (2) ist mit einem Doppelpfeil angedeutet und ist im vorliegenden Fall in einem Winkel von etwa 45° zur Längsachse der Bewegungsrichtung der Bruchbacke (4) ausgerichtet. Die Bewegungsrichtung der Bruchbacke (4) ist in der dargestellten Ausführungsform senkrecht in Richtung der Gegenbacke (3). Die Bewegung des Blechstreifens wird in der vorliegenden Ausführungsformüber eine Gewindespindel (5) ermöglicht. Dadurch kann die Länge des Verfahrweges individuell an jeden Oblong angepasst werden.

Bei dem erfindungsgemäßen Verfahren wird eine Tablette (10), vorzugsweise eine Oblong-Tablette, in die Prüfkammer (1) befördert. Die Tablette kommt durch die Bewegung des Blechstreifens (2) in einer ersten Richtung in Kontakt mit einer ersten Positionierungsfläche, nämlich der Gegenbacke (3), und wird an dieser ausgerichtet. Die Ausrichtung der Tablette (10) erfolgt dadurch, dass diese durch die Bewegung des Blechstreifens (2) in Kontakt mit der Gegenbacke (3) kommt und an der Gegenbacke (3) aufgrund der Bewegung des Blechstreifens (2) entlang ihrer Längsachse ausgerichtet wird. Anschließend wird mindestens ein Parameter der Tablette (10) gemessen. Um eine weitere Prüfung durchzuführen, wird die Tablette (10) zur Prüfung in einer weiteren Position dadurch positioniert, dass der Blechstreifen (2) in einer Richtung entgegengesetzt der ersten Bewegungsrichtung bewegt wird, sodass die Tablette (10) entlang einer weiteren Positionierungsfläche, des Widerlagers (6), ausgerichtet wird.

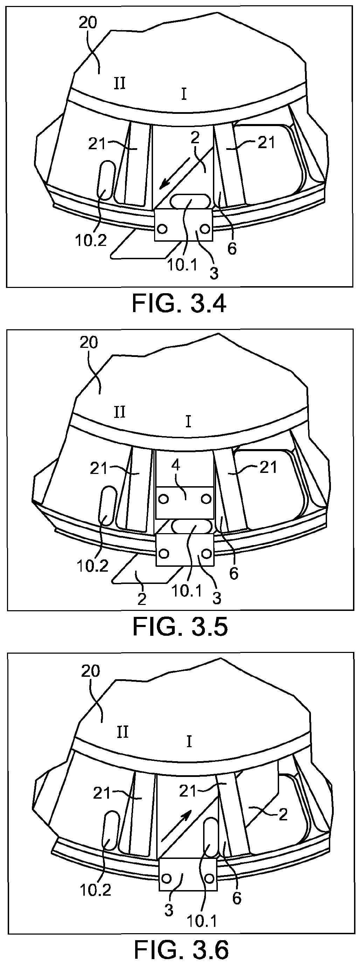

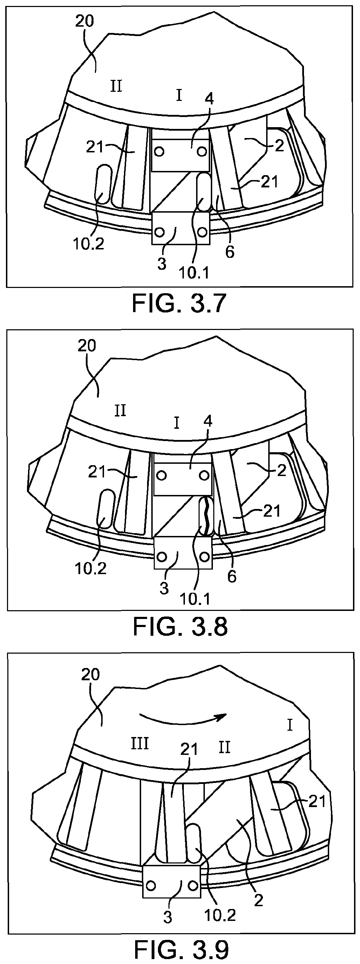

In einer bevorzugten Ausgestaltung der Vorrichtung in **Fig. 2** ist ein Mitnehmer (20) vorgesehen, der das Tablettenmaterial nach erfolgter Prüfung aus der Prüfkammer schiebt. In der vorliegenden Ausführungsform besteht der nach dem Prinzip einer Zellradschleuse arbeitende Mitnehmer (20) aus einem drehbar gelagerten runden Grundkörper und aus mehreren Mitnehmerarmen (21), die sich in radialer Richtung von diesem erstrecken. Der Abstand der Mitnehmerarme (21) entspricht am Übergang zu dem Grundkörper im Wesentlichen der Breite der Bruchbacke (4) (siehe auch Fig. 3.5, 3.7 und 3.8) und in der Prüfposition des Mitnehmers (20) werden die seitlichen Begrenzungen der Prüfkammer (1) jeweils durch zwei benachbarte Mitnehmerarme (21) gebildet. Aufgrund der radialen Anordnung der Mitnehmerarme (21) ergibt sich damit keine rechteckige Prüfkammer (1), sondern eine trapezförmige. Eine Prüfposition des Mitnehmers definiert sich im Sinne dieser Anmeldung dadurch, dass die Bruchbacke (4), die Gegenbacke (3) und jeweils zwei der Arme des Mitnehmers (21) im Wesentlichen trapezförmig zueinander ausgerichtet sind und so die Bewegung der Bruchbacke (4) zwischen den zwei Armen des Mitnehmers (21) ermöglicht wird.

Der Mitnehmer (20) kann die Tablette (10) außerdem vor der Überprüfung in die Prüfkammer, auf den Blechstreifen (2) schieben. Der Mitnehmer (20) kann somit sowohl die Tablette (10) auf den Blechstreifen (2) transportieren, als auch nach erfolgter Prüfung die Tablette (10) oder Tablettenreste von dem Blechstreifen (2) aus der Prüfkammer (1) rausschieben. Die Bewegungsrichtung des Mitnehmers ist mit einem gebogenen Doppelpfeil dargestellt.

Im vorliegenden Fall ist das Widerlager (6) als Teil des Mitnehmers (20) ausgestaltet. Dazu ist an den Mitnehmerarmen (21) jeweils auf der der Drehrichtung beim Einschieben der Tabletten (10) in die Prüfkammer (1) abgewandten Seite ein Widerlager (6) angebracht, wobei der Winkel zwischen dem Widerlager (6) und dem Mitnehmerarm (21) derart gestaltet ist, dass das Widerlager (6) in der Prüfposition des Mitnehmers (20) im Wesentlichen im rechten Winkel zur Gegenbacke (3) steht. In Fig. 2 ist eine Drehrichtung entgegen des Uhrzeigersinns vorgesehen, um die Tablette (10) in die Prüfkammer (1) zu befördern. Dementsprechend sind die Widerlager (6) in Fig. 2 jeweils auf der linken Seite der Mitnehmerarme (21) angeordnet.

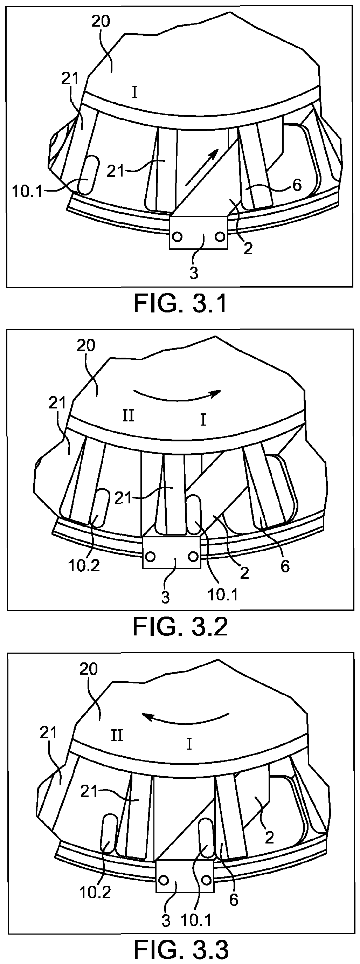

Figuren 3.1 bis 3.9 zeigen einen möglichen Ablauf für das erfindungsgemäße Verfahren zur Prüfung von Tabletten für den Einsatz in einer halbautomatischen Vorrichtung zur Prüfung von Tabletten. Mit den römischen Ziffern I bis III sind die einzelnen Positionen im Mitnehmer beschriftet.

Wie in **Fig. 3****.****1** dargestellt, wird die zu prüfende Tablette (10.1), im vorliegenden Fall eine Oblong-Tablette (10.1) manuell vom Benutzer am Arm (21) des Mitnehmers (20) möglichst nahe am äußeren Radius des Mitnehmers in einer Kammer (Position I) positioniert. Bevor der Oblong (10.1) zur Prüfung in die Prüfkammer auf den Blechstreifen befördert wird, fährt der Blechstreifen (2) in die oberste Position, wobei die Bewegungsrichtung des Blechstreifens (2) durch den Pfeil angedeutet ist. Nach Rotation des Mitnehmers (20) um eine Position (Bewegungsrichtung ist in **Fig. 3****.****2** durch einen gebogenen Pfeil dargestellt), wird der Oblong (10.1) in die Prüfkammer auf den Blechstreifen (2) befördert. Der Mitnehmer (20) wird dabei so weit gedreht, bis die in Drehrichtung vorne liegende Seite (in Fig. 3.2 also die rechte Seite) des die Tablette (10.1) schiebenden Mitnehmerarms (21) mit der in Drehrichtung vorne liegenden Seite der Prüfkammer (1) abschließt (in Fig. 3.2 also ebenfalls deren rechte Seite), sodass die Tablette sich zumindest teilweise außerhalb der Prüfkammer befindet.

Gleichzeitig kann eine weitere zu prüfende Tablette (10.2) in die nächste Kammer des Mitnehmers positioniert werden (Position II).

Danach bewegt sich der Mitnehmer (20) in die entgegengesetzte Richtung zurück (die Bewegungsrichtung ist durch den gebogenen Pfeil dargestellt), wie in **Fig. 3.3** illustriert. Es wird dabei gerade solange gedreht, bis die Tablette (10.1) wieder vollständig innerhalb der Prüfkammer zu liegen kommt. Dies bezweckt, dass der Weg für die Bruchbacke (4) frei wird und die Tablette (10.1) am Widerlager (6) und gegebenenfalls auch an der Gegenbacke (3) anliegt. Das Widerlager (6) ist in dieser Stellung dann auch rechtwinklig zur Gegenbacke (3) und der Bruchbacke (4) ausgerichtet und bildet ein linksseitig offenes Rechteck mit diesen, d. h. es grenzt unmittelbar an deren rechte Ränder an.

In **Fig. 3.4** ist dargestellt, dass durch Bewegung des Blechstreifens (2) die Tablette (10.1) an der Gegenbacke (3) entlang ihrer Längsachse ausgerichtet wird. Die Bewegungsrichtung des Blechstreifens (2), schräg nach unten in Richtung der Gegenbacke (3) ist durch einen Pfeil dargestellt. Bedingt durch die Anordnung der Längsachse des beweglichen Blechstreifens (2) in einem Winkel von unter 90° zur Längsachse der Bewegungsrichtung der Bruchbacke, im vorliegenden Ausführungsbeispiel etwa 45°, kann der Oblong (10.1) an der Gegenbacke (3) ausgerichtet werden. Falls der Oblong (10.1) die Gegenbacke noch nicht kontaktiert, bevor sich der Blechstreifen (2) in Bewegung setzt, kontaktiert der Oblong (10.1) die Gegenbacke, nachdem er durch Bewegung des Blechstreifens (2) gegen die Gegenbacke bewegt wird.

In einem nächsten Schritt, der in **Fig. 3.5** illustriert ist, fährt die Bruchbacke (4) in Richtung der Gegenbacke (3) zwischen den zwei Armen des Mitnehmers (21) hindurch, um einen Parameter des Oblongs (10.1) zu bestimmen. Im vorliegenden Ausführungsbeispiel wird zunächst die Breite des Oblongs (10.1) bestimmt. Nach Vermessung der Breite des Oblongs (10.1), fährt die Bruchbacke wieder zurück in ihre Ausgangsposition.

Um einen weiteren Parameter bestimmen zu können, bewegt sich der Blechstreifen (2) im nachfolgenden Schritt in die entgegengesetzte Richtung, wie in **Fig. 3.6** dargestellt. Die Bewegungsrichtung des Blechstreifens (2) schräg nach oben in Richtung des Widerlagers (6) ist mit einem Pfeil dargestellt. Durch die Bewegung des Blechstreifens (2) in die entgegengesetzte Richtung wird der Oblong (10.1) in Richtung des Widerlagers (6) bewegt und kontaktiert diese Positionierungsfläche. Durch die schräge Anordnung des Blechstreifens (2) wird der Oblong (10.1) durch die Bewegung des Blechstreifens (2) der Länge nach an dem Widerlager (6) ausgerichtet, so dass der Oblong (10.1) um 90° gedreht zu seiner ersten Positionierung zu liegen kommt. Das Widerlager (6) ist als Teil des Mitnehmers (20) ausgestaltet.

Im nachfolgenden Schritt fährt die Bruchbacke (4) wieder in Richtung der Gegenbacke (3), um die Länge des Oblongs (10.1) zu bestimmen. Dies ist in **Fig. 3.7** dargestellt. Nach der Vermessung der Länge des Oblongs (10.1), wird die Bruchhärte bestimmt. Hierzu übt die Bruchbacke (4) eine Kraft in Richtung der Gegenbacke (3) aus, bis der Oblong (10.1) zwischen Bruchbacke (4) und Gegenbacke (3) zerbricht, wie in **Fig. 3.8** zu sehen ist. Durch eine weitere Bewegung des Mitnehmers (20) wird der zerbrochene Oblong entsorgt. Dies ist in **Fig. 3.9** dargestellt. Die Rotationsrichtung des Mitnehmers (20) ist mit dem gebogenen Pfeil dargestellt. Nach Entsorgung des zerbrochenen Oblongs, ist Position I des Mitnehmers geleert und der Oblong aus Position II (10.2) wird in die Prüfkammer auf den Blechstreifen (2) befördert, damit dieser ebenfalls vermessen werden kann. In Position III kann daraufhin ein weiterer Oblong positioniert werden, der in einem nachfolgenden Schritt untersucht werden kann.

### Bezugszeichenliste

- 1: Prüfkammer
- 2: beweglicher Blechstreifen
- 3: Gegenbacke
- 4: Bruchbacke
- 5: Antrieb
- 6: Widerlager
- 10: Tablette/Oblong
- 10.1: Oblong in Position I während des Prüfvorgangs
- 10.2: 2. Oblong in Position II
- 20: Mitnehmer
- 21: Arme des Mitnehmers

## Patentansprüche

1. Vorrichtung zur Prüfung von Tabletten umfassend eine Prüfkammer (1), die eine Bruchbacke (4) und eine ihr gegenüberliegende Gegenbacke (3) aufweist, wobei
- ein Mitnehmer (20) vorgesehen ist, der die zu prüfenden Tabletten in die Prüfkammer und das Tablettenmaterial nach erfolgter Prüfung aus der Prüfkammer schiebt, und der Mitnehmer aus einem drehbar gelagerten runden Grundkörper besteht, von dem sich in radialer Richtung eine Vielzahl von Mitnehmerarmen erstrecken, wobei
- die Vorrichtung über eine Rechenanlage verfügt, welche die Bewegungsabläufe derart steuert, dass ein Mitnehmerarm (21) die Tablette in die Prüfkammer schiebt, während gleichzeitig ein benachbarter Mitnehmerarm das Tablettenmaterial aus der Prüfkammer schiebt, und
- der Abstand der Mitnehmerarme am Übergang zu dem Grundkörper im Wesentlichen der Breite der Bruchbacke entspricht und in der Prüfposition des Mitnehmers die seitlichen Begrenzungen der Prüfkammer jeweils durch zwei benachbarte Mitnehmerarme gebildet werden, und
- die Vorrichtung derart eingerichtet ist, dass das Verschieben der zu prüfenden Tabletten und des Tablettenmaterials durch eine Rotationsbewegung des Mitnehmers erfolgt,
**dadurch gekennzeichnet, dass**
- die Vorrichtung über einen beweglichen Blechstreifen (2) verfügt, der dazu eingerichtet ist, eine Tablette zur Prüfung zu positionieren, und
- die Längsachse des beweglichen Blechstreifens in einem Winkel von unter 90° zur Längsachse der Bewegungsrichtung der Bruchbacke ausgerichtet ist und die Bewegungsrichtung des Blechstreifens der Längsachse des Blechstreifens entspricht,
- die Rechenanlage die Bewegungsabläufe weiterhin derart steuert, dass - der Mitnehmer zunächst so weit in Drehrichtung von der Tablette zur Prüfkammer hin gedreht wird, bis die in Drehrichtung vorne liegende Seite des die Tablette schiebenden Mitnehmerarms mit der in Drehrichtung vorne liegenden Seite der Prüfkammer abschließt, sodass die Tablette sich zumindest teilweise außerhalb der Prüfkammer befindet, und
- der Mitnehmer anschließend so weit in entgegengesetzter Richtung gedreht wird, bis die Tablette wieder vollständig innerhalb der Prüfkammer zu liegen kommt.

2. Vorrichtung nach Anspruch 1, **dadurch gekennzeichnet, dass** in der Prüfkammer ein Widerlager (6) vorgesehen ist, das die Prüfkammer begrenzt und sich von der Gegenbacke in Richtung der Bruchbacke erstreckt, wobei das Widerlager im Wesentlichen in einem rechten Winkel zu der Gegenbacke angeordnet ist.

3. Vorrichtung nach einem der vorherigen Ansprüche, **dadurch gekennzeichnet, dass** die Tablette entlang des Widerlagers durch Bewegung des Blechstreifens in eine Richtung positionierbar ist, und/oder die Tablette entlang der Gegenbacke durch Bewegung des Blechstreifens in die entgegengesetzte Richtung positionierbar ist.

4. Vorrichtung nach einem der vorherigen Ansprüche, **dadurch gekennzeichnet, dass** ein Teil des Blechstreifens einen Boden der Prüfkammer bildet.

5. Vorrichtung nach einem der vorherigen Ansprüche, **dadurch gekennzeichnet, dass** die Bruchbacke dazu geeignet ist, den Durchmesser der Tablette zu messen, die entlang der Gegenbacke oder des Widerlagers ausgerichtet ist, wobei der Durchmesser in Richtung der Bewegungsrichtung der Bruchbacke gemessen wird.

6. Vorrichtung nach einem der vorherigen Ansprüche, **dadurch gekennzeichnet, dass** die Bruchbacke dazu geeignet ist, eine Überprüfung der Bruchhärte einer Tablette durchzuführen, die entlang der Gegenbacke oder des Widerlagers ausgerichtet ist.

7. Vorrichtung nach einem der vorherigen Ansprüche, **dadurch gekennzeichnet, dass** der Winkel zwischen der Längsachse des beweglichen Blechstreifens und der Längsachse der Bewegungsrichtung der Bruchbacke zwischen 20° und 60° beträgt, vorzugsweise zwischen 40° und 50°.

8. Vorrichtung nach einem der vorherigen Ansprüche, **dadurch gekennzeichnet, dass** eine Verstellvorrichtung vorgesehen ist, auf der der Blechstreifen angeordnet ist, wobei mittels der Verstellvorrichtung der Winkel zwischen der Längsachse des beweglichen Blechstreifens und der Längsachse der Bewegungsrichtung der Bruchbacke verstellbar ist.

9. Vorrichtung nach einem der vorherigen Ansprüche, **dadurch gekennzeichnet, dass** an den Mitnehmerarmen jeweils auf der der Drehrichtung beim Einschieben der Tabletten in die Prüfkammer abgewandten Seite ein Widerlager angebracht ist, wobei der Winkel zwischen dem Widerlager und dem Mitnehmerarm derart gestaltet ist, dass das Widerlager in der Prüfposition des Mitnehmers im Wesentlichen im rechten Winkel zur Gegenbacke steht.

10. Verfahren zur Prüfung von Tabletten, wobei
- ein Mitnehmer (20) die zu prüfenden Tabletten in eine Prüfkammer (1) und das Tablettenmaterial nach erfolgter Prüfung aus der Prüfkammer schiebt und das Verschieben der zu prüfenden Tabletten und des Tablettenmaterials durch eine Rotationsbewegung des Mitnehmers erfolgt, und
- ein Mitnehmerarm (21) die Tablette in die Prüfkammer schiebt während gleichzeitig ein benachbarter Mitnehmerarm das Tablettenmaterial aus der Prüfkammer schiebt,
**dadurch gekennzeichnet, dass**
- eine Tablette zur Prüfung auf einen beweglichen Blechstreifen (2) aufgebracht wird und die Tablette durch Bewegung des Blechstreifens entlang mindestens einer Positionierungsfläche ausgerichtet wird und anschließend mindestens ein Parameter der Tablette gemessen wird,
- der Mitnehmer zunächst so weit in Drehrichtung von der Tablette zur Prüfkammer hin gedreht wird, bis die in Drehrichtung vorne liegende Seite des die Tablette schiebenden Mitnehmerarms mit der in Drehrichtung vorne liegenden Seite der Prüfkammer abschließt, sodass die Tablette sich zumindest teilweise außerhalb der Prüfkammer befindet, und
- der Mitnehmer anschließend so weit in entgegengesetzter Richtung gedreht wird, bis die Tablette wieder vollständig innerhalb der Prüfkammer zu liegen kommt.

11. Verfahren nach Anspruch 10, **dadurch gekennzeichnet, dass** zur Prüfung der Tablette in einer ersten Position diese dadurch positioniert wird, dass der bewegliche Blechstreifen in eine ersten Richtung bewegt wird, sodass die Tablette entlang einer Gegenbacke (3) ausgerichtet wird und/oder die Tablette zur Prüfung in einer weiteren Position dadurch positioniert wird, dass der bewegliche Blechstreifen in eine Richtung entgegengesetzt der ersten bewegt wird, sodass die Tablette entlang eines Widerlagers (6) ausgerichtet wird.

12. Verfahren nach einem der Ansprüche 10 und 11, **dadurch gekennzeichnet, dass** die Prüfung der Tablette durch eine Bruchbacke (4) durchgeführt wird, die eine Bewegung in Richtung der Gegenbacke vollzieht.

13. Verfahren nach einem der Ansprüche 10 bis 12, **dadurch gekennzeichnet, dass** die Bruchbacke eine Prüfung des Durchmessers der Tablette durchführt, die entlang der mindestens einen Positionierungsfläche ausgerichtet ist, indem sie eine Bewegung in Richtung der Gegenbacke so lange vollzieht, bis auf die Bruchbacke eine Gegenkraft gemäß einem vordefinierten Wert auftritt.

14. Verfahren nach einem der Ansprüche 10 bis 13, **dadurch gekennzeichnet, dass** die Bruchbacke eine Prüfung der Bruchhärte einer Tablette durchführt, die entlang der mindestens einen Positionierungsfläche ausgerichtet ist, indem sie eine Bewegung in Richtung der Gegenbacke so lange vollzieht, bis auf die Bruchbacke eine Gegenkraft gemäß einem vordefinierten Wert auftritt, woraufhin die durch die Bruchbacke angelegte Kraft in Bewegungsrichtung so lange gesteigert wird, bis ein Bruch der Tablette auftritt.

15. Verfahren nach einem der Ansprüche 10 bis 14, **dadurch gekennzeichnet, dass** die Mitnehmerarme jeweils auf der der Drehrichtung beim Einschieben der Tabletten in die Prüfkammer abgewandten Seite mit einem Widerlager (6) versehen sind und das Zurückdrehen des Mitnehmers so weit erfolgt, bis das Widerlager im Wesentlichen im rechten Winkel zu der Bruchbacke und der Gegenbacke steht und das Widerlager mit diesen im Wesentlichen ein offenes Rechteck bildet.

## Claims

1. Device for testing of tablets, comprising a test chamber (1), featuring
a crushing jaw (4) and a counter jaw (3) opposite it,
whereby
- a conveyer (20)
is arranged to position the tablets to be tested into the test chamber and pushing the tablet material back from the test chamber after testing, and the conveyor consisting of a pivoted round base body, from which several driver arms extending in a radial direction, whereby
- the device featuring a computer system, controlling the motion sequences in such a way that one driver arm (21) pushing the tablet into the test chamber, while one adjacent driver arm pushing the tablet material from the test chamber at the same time, and
- the distance of the driver arms at the passage to the base body basically corresponds to the breadth of the crushing jaw and the lateral boundary of the test chamber are each respectively formed by two adjacent driver arms in the testing position, and
- the device being arranged in such a way that moving the tablets to be tested as well as the tablet material takes place through a rotation movement of the driver,
**characterised in that**
- the device featuring a movable sheet metal (2) arranged to position a tablet for testing and
- the longitudinal axis of the moving sheet metal strip is oriented at an angle of less than 90 °C to the longitudinal axis of the direction of movement of the breaking jaw and the direction of movement of the metal strip corresponding to the longitudinal axis of the sheet metal strip,
- the computer system still controlling the motion sequences in such a way that the conveyer is turned in the direction of rotation from the tablet to the test chamber until the side, situated in front and in direction of rotation of the tablet pushed by the driving arm in direction of rotation, in such a way that the tablet is at least partly situated outside of the test chamber, and
- the conveyer subsequently turned in the opposite direction so far until the tablet is finally fully positioned inside the test chamber again.

2. Device according to claim 1, **characterised in that** an abutment is situated in the test chamber (6) restricting the test chamber and extending from the counter jaw into the direction of the crushing jaw, whereby the abutment is basically set at a right angle in respect to the counter jaw.

3. Device according to one of the previous claims, **characterised in that** the tablet is positionable by moving the sheet metal in a direction alongside the abutment and/or the tablet is positionable alongside the counter jaw by moving the sheet metal into the opposite direction.

4. Device according to one of the previous claims, **characterised in that** a part of the sheet metal forming a bottom of the test chamber.

5. Device according to one of the previous claims, **characterised in that** the crushing jaw is suitable to measure the diameter of the tablet, which is positioned alongside the counter jaw or the abutment, whereby the diameter is measured in the direction of the movement of the crushing jaw.

6. Device according to one of the previous claims, **characterised in that** the crushing jaw is suitable to carry out an inspection of the crushing strength of a tablet, which is directed alongside the counter jaw or the abutment.

7. Device according to one of the previous claims, **characterised in that** the angle between the longitudinal axis of the movable sheet metal and the longitudinal axis of the crushing jaw lies between 20 °C and 60 °C, preferably between 40 °C and 50 °C.

8. Device according to one of the previous claims, **characterised in that** an adjusting advice is arranged on which the metal sheet is arranged, whereby the angle between the longitudinal axis of the movable metal sheet and the longitudinal axis of the direction of movement of the crushing jaw is adjustable.

9. Device according to one of the previous claims, **characterised in that** an abutment is mounted at the driver arms, each on the direction of rotation when inserting the tablet into the test chamber on the side facing away from the side of the chamber, whereby the angle between the abutment and the driver arm is designed in such a shape that the abutment in the test position of the conveyer is basically at a right angle in respect to the counter jaw.

10. Method for testing tablets, whereby
- a conveyer (20) is pushing the tablets to be tested into a test chamber (1) and pushing
the tablet material back from the test chamber after testing by means of a rotational movement of the conveyer and a
- driving arm (21)
pushing the tablet into the test chamber, while at the same time an adjacent driver arm pushing the tablet material out of the test chamber,
**characterised in that**
- a tablet is mounted on a movable sheet metal (2) for testing, the tablet being aligned by moving the sheet metal alongside at least one positioning surface and subsequently at least one parameter of the tablet being measured.
- the conveyer
that the conveyer is turned in the direction of rotation from the tablet to the test chamber until the side situated in front and in direction of rotation of the tablet pushed by the driving arm in direction of rotation, in such a way that the tablet is at least partly situated outside of the test chamber, and
- the conveyer subsequently turned in the opposite direction so far until the tablet is finally positioned inside the test chamber again.

11. Method according to claim 10, **characterised in that** for testing the tablet in a first position, this one is positioned by moving the movable sheet metal in a first direction, allowing the tablet to be positioned alongside the counter jaw (3) and/or the tablet for testing in another position to be positioned by moving the movable sheet metal in a direction opposite the first one, enabling the tablet to be positioned alongside the abutment (6).

12. Method according to one of the claims 10 and 11, **characterised in that** the testing being pursued by means of a crushing jaw (4), carrying out a movement into the direction of the counter jaw.

13. Method according to one of the claims 10 to 12, **characterised in that** the crushing jaw is able to measure the diameter of the tablet, which is arranged alongside at least one positioning surfaces, by means of carrying out a movement into the direction of the counter jaw, until a counterforce onto the crushing jaw occurs, corresponding to a predefined value.

14. Method according to one of the claims 10 to 13, **characterised in that** the crushing jaw is able to measure the crushing strength of the tablet, which is arranged alongside at least one positioning surfaces, by means of carrying out a movement into the direction of the counter jaw, until a counterforce onto the crushing jaw occurs, corresponding to a predefined value
whereafter the power in direction of movement, induced by the crushing jaw is being increased until the tablet breaks.

15. Method according to one of the claims 10 to 14, **characterised in that** the driving arms are equipped with an abutment (6), each on the direction of rotation when inserting the tablet into the test chamber on the side facing away from the side of the chamber, turning back the conveyer is carried out until the abutment is basically at a right angle in respect to the crushing jaw and the counter jaw, the abutment basically forming an open rectangle together with the crushing jaw and the counter jaw.

## Revendications

1. Dispositif pour le contrôle des comprimés comprenant une chambre d'essai (1), laquelle présente une mâchoire de fracture (4) et une contre-mâchoire opposante (3),
où
- une douille d'accouplement (20)
est prévue, laquelle achemine le comprimé à tester à l'intérieur de la chambre d'essai, retirant la matière du comprimé de la chambre d'essai après l'accomplissement de son testage. Cette douille d'accouplement est constituée d'un corps de base ronde pivotant, à partir duquel plusieurs bras s'étendent radialement.
- le dispositif est connecté à un système informatique, lequel contrôle l'exécution de la séquence des mouvements de façon à ce que le bras GIN de la douille d'accouplement (21) achemine le comprimé à l'intérieur la chambre d'essai, pendant que, simultanément, un bras avoisinant retire la matière du comprimé de la chambre d'essai.
- l'écart entre la jonction et le corps de base de la douille d'accouplement correspond en gros à la largeur de la mâchoire de fracture. Quand en position de test, deux bras adjacents de la douille d'accouplement définissent les limites latérales de la chambre d'essai.
- le dispositif est configuré de telle sorte que le déplacement des comprimés à tester et de la matière des comprimés s'effectuent par un mouvement de rotation de la douille d'accouplement
**caractérisé en ce que :**
- le dispositif est transporté sur une glissière métallique mouvante (2), laquelle
- est destinée à positionner le comprimé pour le test,
- son axe longitudinal est orienté à un angle inférieur à 90 degrés par rapport à l'axe longitudinal de la direction de mouvement de la mâchoire de fracture, la glissière métallique se déplaçant sur son axe longitudinal,
- le système informatique contrôle la séquence des mouvements de façon à ce que la douille d'accouplement d'abord renverse le sens de rotation du comprimé dedans la chambre d'essai, jusqu'à ce que, dans cette direction de mouvement, le côté situé en avant du bras de la douille d'accouplement qui achemine le comprimé, et le côté situé en avant de la chambre d'essai, soient parallèles entre eux. De cette façon, le comprimé sera au moins partiellement à l'extérieur de la chambre d'essai,
- ensuite la douille d'accouplement tourne dans le sens opposé, jusqu'à ce que le comprimé soit à nouveau entièrement à l'intérieur de la chambre d'essai

2. le dispositif selon la revendication 1, **caractérisé en ce que** une butée (6) est utilisée à l'intérieur la chambre d'essai,
laquelle délimite la chambre d'essai,
tout en s'étendant de la contre-mâchoire à la mâchoire, de façon que la butée est perpendiculaire à la contre-mâchoire.

3. Le dispositif selon l'une quelconque des revendications précédentes, **caractérisé en ce que** les comprimés sont orientés par le biais du mouvement de la glissière métallique le long de la butée, et/ou que les comprimés sont orientés par le mouvement de la glissière métallique dans le sens opposé, le long de la contre-mâchoire.

4. Le dispositif selon l'une quelconque des revendications précédentes, **caractérisé en ce qu'**une partie de la glissière métallique forme un fond pour la chambre d'essai.

5. Le dispositif selon l'une quelconque des revendications précédentes, **caractérisé en ce que** la mâchoire de fracture mesure le diamètre du comprimé avec ceci orienté le long de la butée ou de la contre-mâchoire, de façon que le diamètre du comprimé est mesuré dans le sens du mouvement de la mâchoire de fracture.

6. Le dispositif selon l'une quelconque des revendications précédentes, **caractérisé en ce que** la mâchoire de fracture effectue le test de dureté du comprimé avec ceci orienté le long de la contre-mâchoire ou de la butée.

7. Le dispositif selon l'une quelconque des revendications précédentes, **caractérisé en ce que** l'angle entre l'axe longitudinal de la glissière métallique mouvante et l'axe longitudinal de la direction du mouvement de la mâchoire de fracture est fixé entre 20 et 60 degrés, et de préférence entre 40 et 50 degrés.

8. Le dispositif selon l'une quelconque des revendications précédentes, **caractérisé en ce qu'**un dispositif de réglage au-dessus duquel la glissière métallique est disposée, règle l'angle entre l'axe longitudinal de la glissière métallique mouvante et l'axe longitudinal de la direction de mouvement de la mâchoire de fracture.

9. Le dispositif selon l'une quelconque des revendications précédentes, **caractérisé en ce que** dans chaque bras de la douille d'accouplement une butée est utilisée dans le côté opposé à celui du mouvement par lequel le comprimé est acheminé à l'intérieur de la chambre d'essai. L'angle entre la butée et le bras de la douille d'accouplement doit être réglé de façon que, quand la douille d'accouplement est en position de test, la butée reste essentiellement perpendiculaire à la contre-mâchoire.

10. Procédé de contrôle des comprimés, où
- une douille d'accouplement (20) achemine le comprimé à tester à l'intérieur la chambre d'essai (1),
et retire la matière du comprimé de la chambre d'essai après l'accomplissement de son testage. Le déplacement du comprimé à tester et de la matière du comprimé s'effectue par un mouvement de rotation de la douille d'accouplement, où un bras de la douille d'accouplement (21)
achemine le comprimé à l'intérieur de la chambre d'essai pendant que, simultanément, un autre bras avoisinant retire la matière du comprimé de la chambre d'essai.
**caractérisé en ce que**,
- une glissière métallique (2) soit utilisé dans le test du comprimé et
**que** le comprimé à tester soit orienté, par le biais du mouvement de cette glissière métallique, le long d'au moins une surface de positionnement, à la suite de quoi au moins un paramètre du comprimé est mesuré,
- la douille d'accouplement renverse le sens de rotation du comprimé à l'intérieur de la chambre d'essai, jusqu'à ce que le côté situé en avant du bras de la douille d'accouplement qui achemine le comprimé, et le côté situé en avant de la chambre d'essai, soient parallèles entre eux. De cette façon, le comprimé sera au moins partiellement à l'extérieur de la chambre d'essai, et
- ensuite la douille d'accouplement tourne dans le sens opposé jusqu'à ce que le comprimé soit, encore une fois, entièrement à l'intérieur de la chambre d'essai.

11. Procédé selon la revendication 10, **caractérisé en ce que** le test du comprimé s'effectue d'abord dans une position initiale, où la glissière métallique mouvante se déplace dans une direction permettant l'orientation du comprimé le long de la contre-mâchoire (3) et/ou le comprimé à tester soit positionné dans une autre position, où la glissière métallique mouvante se déplace dans une direction opposée à celle première, de façon que le comprimé soit orienté le long de la butée (6).

12. Procédé selon l'une quelconque des revendications 10 ou 11, **caractérisé en ce que** le test du comprimé s'effectue par le biais d'une mâchoire de fracture (4) laquelle est mue dans la direction de la contre-mâchoire.

13. Procédé selon l'une quelconque des revendications 10 à 12, **caractérisé en ce que** la mâchoire de fracture effectue un test sur le diamètre du comprimé, lequel est aligné le long d'au moins une surface de positionnement. La mâchoire de fracture est mue dans la direction de la contre-mâchoire, et en exerce une force antagoniste en conformité avec une valeur préétablie.

14. Procédé selon l'une quelconque des revendications 10 à 13, **caractérisé en ce que** la mâchoire de fracture effectue un test de dureté du comprimé, lequel est orienté le long d'au moins une surface de positionnement. La mâchoire de fracture est mue dans la direction de la contre-mâchoire, et en exerce une force antagoniste en conformité avec une valeur préétablie. La force appliquée dans le sens du mouvement s'éleve jusqu'à induire une fracture dans le comprimé.

15. Procédé en conformité avec l'une quelconque des revendications 10 à 14, **caractérisé en ce que** les bras de la douille d'accouplement ont chacun une butée (6), lesquelles sont positionnés dans le côté opposé à la direction de l'acheminement du comprimé à l'intérieur la chambre d'essai.
Le retour en arrière de la douille d'accouplement s'accomplit avec la butée perpendiculaire à la mâchoire de fracture et la contre-mâchoire, formant avec ceux-ci la figure d'un rectangle ouvert.
